# EUROPEAN PATENT SPECIFICATION

(11) **EP 2 723 850 B1**
(45) Date of publication and mention of the grant of the patent: **06.04.2016**
(21) Application number: 12725468.8
(22) Date of filing: 05.06.2012
(51) Int. Cl.: C12N 5/00

(54) **METHOD FOR CULTIVATING CELLS IN PLATELET-LYSATE-CONTAINING MEDIUM**
VERFAHREN ZUR KULTIVIERUNG VON ZELLEN IN EINEM MEDIUM MIT THROMBOZYTENLYSAT
PROCÉDÉ DE CULTURE DE CELLULES DANS UN MILIEU CONTENANT UN LYSAT DE PLAQUETTES

(30) Priority: 27.06.2011 EP 11171595
(43) Date of publication of application: 30.04.2014
(73) Proprietor: Rheinisch-Westfälische Technische Hochschule (RWTH) Aachen, 52056 Aachen (DE)
(72) Inventor: WAGNER, Wolfgang, 52078 Aachen (DE); WALENDA, Gudrun, 52064 Aachen (DE)
(74) Representative: Remus, Alvaro Johannes
(86) International application number: PCT/EP2012/060597
(87) International publication number: WO 2013/000672

(56) References cited:
- WO-A1-89/10398
- WO-A1-2006/062989
- LANGE C ET AL: "Accelerated and safe expansion of human mesenchymal stromal cells in animal serum-free medium for transplantation and regenerative medicine", JOURNAL OF CELLULAR PHYSIOLOGY, vol. 213, no. 1, 1 October 2007 (2007-10-01), pages 18-26, XP002545733, WILEY LISS, NEW YORK, NY, US ISSN: 0021-9541, DOI: 10.1002/JCP.21081 [retrieved on 2007-04-25]
- SALVADE AGNESE ET AL: "Characterization of Platelet Lysate Cultured Mesenchymal Stromal Cells and Their Potential Use in Tissue-Engineered Osteogenic Devices for the Treatment of Bone Defects", TISSUE ENGINEERING PART C-METHODS, vol. 16, no. 2, April 2010 (2010-04), pages 201-214, XP002663438, ISSN: 1937-3384
- BLANDE IVAN SOUZA ET AL: "Adipose tissue mesenchymal stem cell expansion in animal serum-free medium supplemented with autologous human platelet lysate", TRANSFUSION, vol. 49, no. 12, December 2009 (2009-12), pages 2680-2685, XP002663439, ISSN: 0041-1132
- HECKMANN LESLIE ET AL: "Interactive effects of growth factors and three-dimensional scaffolds on multipotent mesenchymal stromal cells", BIOTECHNOLOGY AND APPLIED BIOCHEMISTRY, vol. 49, no. Part 3-4, April 2008 (2008-04), pages 185-194, XP002663440, ISSN: 0885-4513

## Description

### Background of the invention

The invention relates to a method for cultivating adherent cells using a liquid medium comprising blood platelet lysate and a gelatinization-inhibiting substance. The invention further relates to a two-phase system and a gel for cultivating cells.

### Prior art

Cells are usually cultured in media that are supplemented with fetal calf serum (FCS) or other blood sera in order to provide the cells with essential growth factors. Although not any cell type actually needs serum for growth, most of the sensitive cell types, e.g., stem cells, are by now still dependent on serum-containing media. However, in light of therapeutic applications there is a growing interest to avoid the use of FCS due to potential xenogeneic immune reactions (e.g. anti-FCS antibodies), bovine pathogens (viruses and prions) and a high lotto-lot variability that hampers reproducibility of results (Heiskanen et al., 2007; Sundin et al., 2007). Chemically defined synthetic culture media are now commercially available, but their precise composition is often undisclosed and they often include other xenogeneic components such as recombinant growth factors.

Human platelet lysate (HPL) represents another attractive alternative to FCS. HPL can be generated from common platelet units by a simple freeze-thaw procedure. It is even possible to use HPL from the same donor to further minimize the risk of immunological side effects or viral infections. These studies were performed with mesenchymal stromal cells (MSC) from bone marrow and recently it has been demonstrated that HPL can also be used for isolation of MSC from human adipose tissue (Blande et al., 2009).

A method for preparing blood platelet lysate and a HPL medium is known from EP 0 413 727 B1. According to this known method citrate is added to whole blood in order to avoid coagulation. Plasma derived from this blood is centrifuged so as to obtain a platelet-rich paste and Ca²⁺ is then added for lysing the platelets and coagulating the remaining components. The filtered clear lysate is mixed with a conventional nutrient composition so as to obtain a HPL-containing medium.

Furthermore, WO 2008/034803 A1 discloses a cell culture medium supplement comprising plasma-free platelet lysate. The supplement further comprises additional substances such as albumin and dextran. In order to prepare the supplement, platelets are lysed by freezing and thawing them and acid citrate dextrose (ACD) or citrate phosphate dextrose (CPD) is added as anti-coagulant.

However, many cells, e.g., mesenchymal stromal cells (MSC), grow in a plastic adherent monolayer until they are contact inhibited. Sooner or later, the proliferation rate of the cells decays until they ultimately reach a senescent state. This is accompanied by enlarged morphology, reduced expression of surface markers and decreased differentiation potential. This process does not occur in parallel across cells in culture due to different subpopulations, apoptosis, and last but not least, contact inhibition within colonies. Lower seeding density facilitates higher intervals for cell passaging and hence, proliferation can only occur at the rim of the larger colonies. Consequently, when using plastic adherent monolayer cultures, it is hardly possible to accomplish a reliable expansion and to minimize contact inhibition. Moreover, cell passaging is not possible without using a cell-damaging peptidase treatment either. With stem cell cultures, it is another disadvantage of plastic adherent monolayer cultures that the plastic surface may cause undesired differentiation of the cells.

### Summary of the invention

It is the object of the invention to provide a method for cultivating adherent cells and a cell culture substrate that allows reliable expansion of cells, wherein contact inhibition is minimized and cell passaging is possible without needing an enzymatic treatment.

The object is achieved by providing a method comprising cultivating the cells on the surface of and/or within a three-dimensional, gel-like substrate comprising 1-40 % (volume concentration) blood platelet lysate (BPL) but being substantially devoid of a gelatinization-inhibiting substance, wherein a liquid medium comprising blood platelet lysate and a gelatinization-inhibiting substance is layered on the gel-like substrate. Gelatinization of pure blood platelet lysate or a composition comprising blood platelet lysate, which is free of any gelatinization-inhibiting substance such as heparin, occurs spontaneously at approximately 37 °C within one hour. Surprisingly, addition of coagulation-inducing agents, such as thrombin and/or calcium gluconate, is not necessary for gelatinization. Moreover, it turned out that the resulting gel-like substrate is a perfect substrate for expanding adherent cells, especially mesenchymal stromal cells (MSC) or fibroblasts. The use of a gel-like substrate comprising blood platelet lysate avoids the risk of xenogenic immunoreactions or transmission of bovine pathogens and is also more efficient than FCS for large-scale expansion of cells, especially MSC from adipose tissue. For example, human platelet lysate (HPL) significantly increases the proliferation rate and maximal number of cumulative population doublings of adipose tissue derived MSC. Thus, the method according to the invention enables reliable expansion of adherent cells and efficient minimization of contact inhibition as the cells can grow three-dimensionally within the substrate, i.e. multilayered, while growth factors are evenly available in all directions. Moreover, cell passaging is possible without the need of an enzymatic treatment, for example, a Trypsin/EDTA digestion, and a centrifugation step. As the gel-like substrate is biocompatible and hence provides a physiological environment for the cells, the risk of any undesired influence on the cells is minimized. For example, undesired differentiation of stem cells can be efficiently avoided. Further, covering the surface of the gel-like substrate with a liquid medium comprising blood platelet lysate and a gelatinization-inhibiting substance advantageously provides a homogenous environment for the cells and thus ensures optimal cultivation conditions.

In a preferred embodiment of the invention, the liquid medium and the gel-like substrate, beside the gelatinization-inhibiting substance, have a substantially identical composition, so that growth factors and nutrients are evenly distributed within the whole cultivation system and even the cells on the surface of the gel-like substrate can be cultivated under homogenous conditions.

In one embodiment of the invention, passaging or harvesting the cells is accomplished by enzymatic degradation of the gel-like substrate, preferably using a peptidase, in particular plasmin. Alternatively, passaging or harvesting the cells may be accomplished by mechanical fragmentation of the gel-like structure, preferably by pipetting, optionally followed by Trypsin/EDTA treatment or EDTA treatment for singularization of the cells. However, as even simple pipetting without any enzymatic treatment is sufficient with the method according to the invention, use of any enzymes during cell passaging can be avoided.

Preferably, the gelatinization-inhibiting substance comprises heparin or a low molecular weight heparin (LMWH) such as Clexane^{®} (Sanofi-Aventis, Paris, France). In a preferred embodiment of the invention, the liquid medium comprises 0.1-100 U/ml Heparin as gelatinization-inhibiting substance, preferably 0.1-10 U/ml, in particular 0.2-5 U/ml. If Clexane^{®} is used, this LMWH may preferably be provided at a concentration of 0.001-1 mg/ml, preferably 0.005 -1 mg/ml, in particular 0.005-0.5 mg/ml.

In another preferred embodiment, both the liquid medium and the gel-like substrate are substantially devoid of serum so as to avoid any xenogeneic immune reactions or pathogenic infections. Thus, the method according to the invention is suitable for providing cells for therapeutic applications.

Preferably, the cells are stem or progenitor cells, particularly preferred mesenchymal stem cells, in particular mesenchymal stromal cells. In another preferred embodiment of the invention, the cells are mesodermal cells, preferably fibroblasts or mesenchymal stromal cells. But, obviously, the method according to the invention may also be suitable for cultivating other types of cells, for example cells derived from ectodermal or endodermal lineages. Also, the method according to the invention is well suited for cultivating primary cells.

The object is further achieved by a method for producing a three-dimensional, gel-like substrate, said method comprising:
a) Providing a thrombocyte concentrate;
b) Harvesting blood platelets from said concentrate;
c) Lysing the blood platelets;
d) Adding at least one medium component to the lysate so as to obtain a medium comprising 1-40 % blood platelet lysate; and
e) Allowing the blood-platelet-lysate-medium to gelatinize at approximately 37 °C so as to obtain a gel-like substrate comprising blood platelet lysate.

Surprisingly, it turned out that blood platelet lysate, either pure or diluted, spontaneously gelatinizes at approximately 37 °C without the necessity to add any coagulation-inducing agent such as, for example, thrombin and/or calcium gluconate.

In a preferred embodiment of this method, the medium component comprises a standardized medium composition for cell culture, preferably Dulbecco's Modified Eagles medium (DMEM), in particular DMEM low glucose (DMEM-LG). Thus, a medium containing essential growth factors and all necessary nutrients for cell cultures without undesired serum components is provided.

According to a preferred embodiment of the invention, the substrate may comprise 5-40 % blood platelet lysate, more preferred 5-20 % blood platelet lysate, in particular approximately 10-20 % blood platelet lysate. For example, the gel-like substrate according to the invention may essentially consist of 10 % HPL, 89 % DMEM, and 2mM L-Glutamine.

The object is further achieved by a two-phase system comprising (a) a liquid medium comprising blood platelet lysate and a gelatinization-inhibiting substance and (b) a three-dimensional, gel-like substrate comprising 1-40 % blood platelet lysate but being substantially devoid of a gelatinization-inhibiting substance.

In a preferred embodiment of this two-phase system, the liquid medium and the gel-like substrate, beside the gelatinization-inhibiting substance, have a substantially identical composition. Preferably, the gelatinization-inhibiting substance in the liquid phase of the two-phase system comprises heparin.

The invention is further described in detail with reference to the figures.

### Brief description of the figures

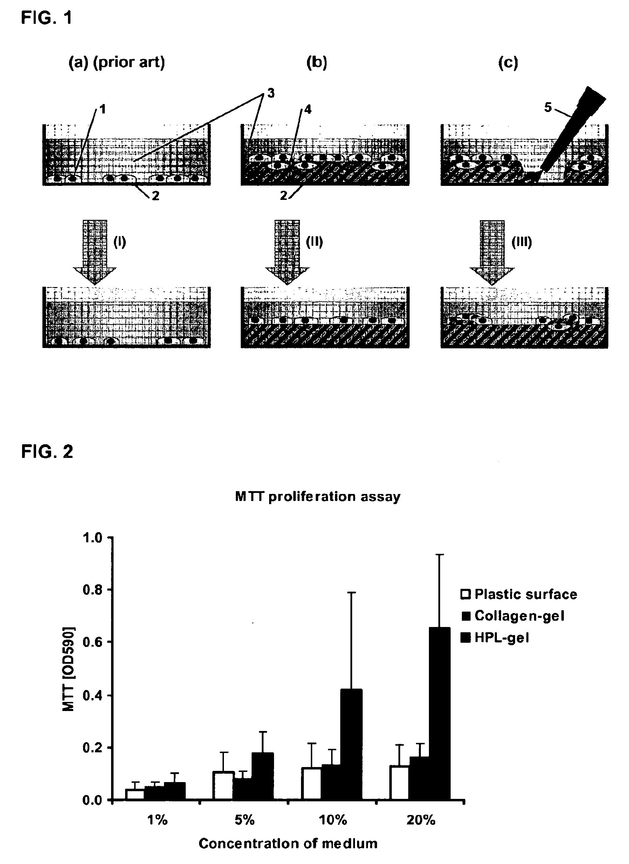

**Figure 1** shows a schematic representation of experimental arrangements for demonstrating the invention. Cells are cultivated either conventionally directly on cell culture plastics or on a gel according to the invention comprising human platelet lysate (HPL) but being devoid of heparin. The liquid culture medium contains essentially identical components but additionally includes heparin for inhibiting coagulation. The cells can be harvested by degradation of the HPL gel using plasmin or by pipetting.
**Figure 2** shows a bar diagram demonstrating the proliferation of mesenchymal stromal cells (MSCs) on different surfaces (white bars: plastic surface; grey bars: collagen gel; black bars: HPL gel). MSCs were cultured in culture medium comprising different concentrations of human platelet lysate (HPL) and the number of cells was determined after seven days using a MTT assay. The HPL concentration within the gel was respectively consistent with the HPL concentration in the liquid medium. Cell proliferation in HPL gel compared to proliferation on plastics and collagen gel shows significant differences (n=5).

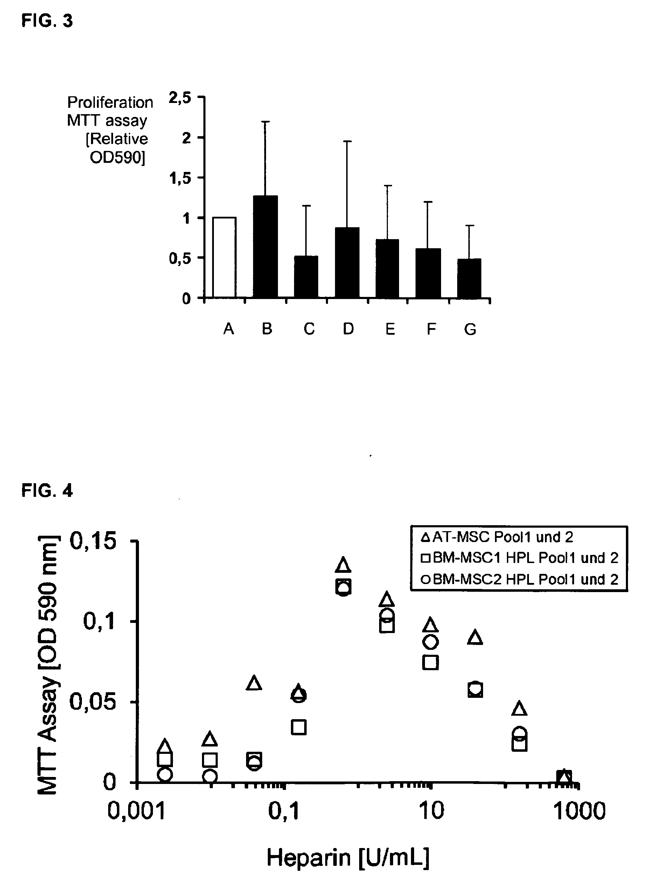

**Figure 3** shows a bar diagram demonstrating the proliferation of mesenchymal stromal cells (MSC) for different cell passaging methods with gel comprising human platelet lysate (HPL). MSC were cultivated for seven days on HPL gels and, for the sake of comparison, on cell culture plastics. Cells were harvested using plasmin treatment or pipetting, with or without subsequent Trypsin/EDTA or EDTA treatment, respectively, and then transferred to a new cell culture dish containing medium without HPL. After one further day of culturing, the number of cells was determined by a MTT assay. For the sake of control, the resulting values (black bars) were normed on cell culture plastic values (white bar, OD590 = 1). N=3, standard deviation is indicated.
   - A:: Cultivation on plastics, passaging by Trypsin/EDTA
   - B:: HPL gel, passaging by pipetting
   - C:: HPL gel, passaging by pipetting and EDTA treatment
   - D:: HPL gel, passaging by pipetting and Trypsin/EDTA treatment
   - E:: HPL gel, passaging by plasmin degradation of the gel
   - F:: HPL gel, passaging by plasmin degradation and EDTA treatment
   - G:: HPL gel, passaging by plasmin degradation and Trypsin/EDTA treatment
**Figure 4** shows a diagram illustrating the proliferation of mesenchymal stromal cells (MSC) derived from bone marrow (BM) or adipose tissue (AT) as a function of Heparin concentration ([U/ml]) within the liquid medium. MSCs were cultivated for seven days on HPL gels covered with liquid HPL medium containing different amounts of Heparin. The number of cells was determined after seven days using a MTT assay (OD 590 nm).

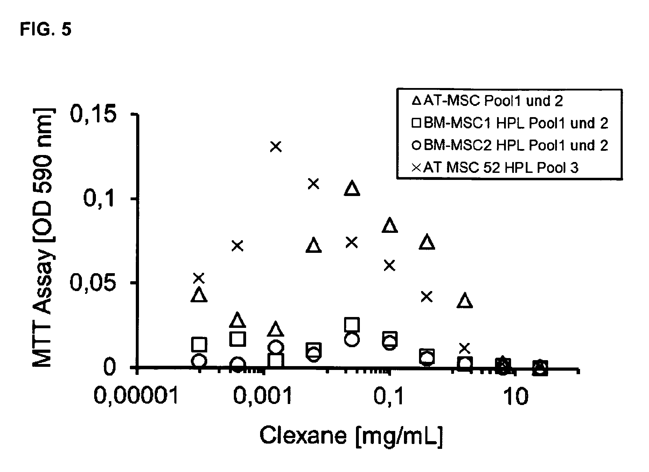

**Figure 5** shows a diagram illustrating the proliferation of mesenchymal stromal cells (MSC) derived from bone marrow (BM) or adipose tissue (AT) as a function of Clexane^{®} (low molecular weight heparin (LMWH) of Sanofi-Aventis, Paris, France) concentration ([mg/ml]) within the liquid medium. MSCs were cultivated for seven days on HPL gels covered with liquid HPL medium containing different concentrations of LMWH. The number of cells was determined after seven days using a MTT assay (OD 590 nm).

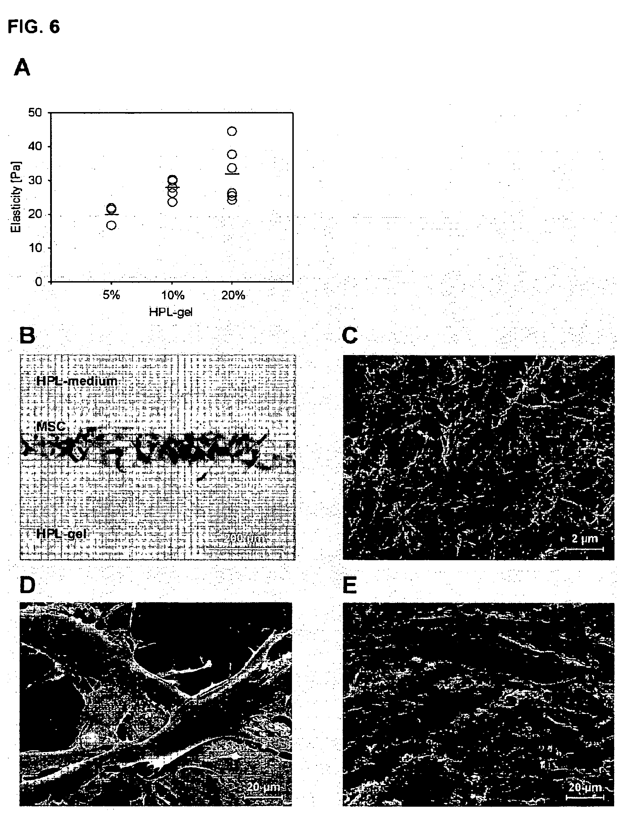

**Figure 6** shows a diagram representing the elasticity of HPL gels as a function of HPL concentration (A) and micrographs demonstrating that MSC grow at the interface of HPL gel and HPL medium (B-E). Human platelet lysate containing cell culture medium without gelatinization-inhibiting substance (anticoagulant) was gelatinized in thin bottom cell culture wells at indicated concentrations. Each HPL concentration was analyzed for elasticity on two independent samples with one to three measurements per sample. The substrate elasticity of HPL-gel was very low and correlated with HPL concentrations (A). Mean elasticity values are indicated as thin line. Then, MSC were seeded on top with the same culture medium supplemented with heparin. Histological sections after 4 days of culture revealed multilayered cell growth at the interface of HPL gel and HPL medium (B). Scanning electron microscopy (SEM) demonstrated the reticular fibrin scaffold of HPL gel (C). MSC on TCP (D) and on HPL-gel (E) showed the typical spindle-shaped MSC morphology.

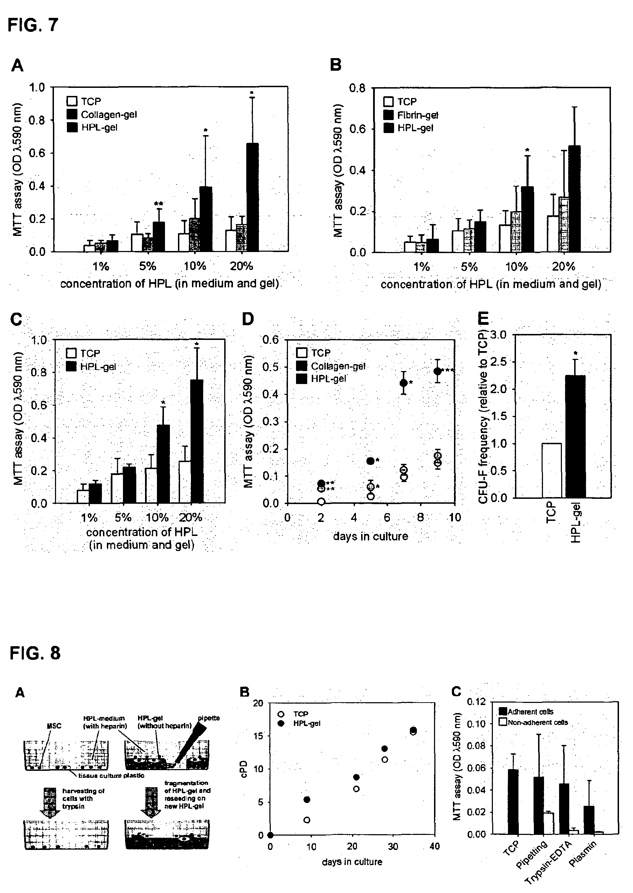

**Figure 7** shows diagrams demonstrating the proliferation rate and CFU-f frequency increase on HPL gel. MSCs were seeded in 96-well plates either on TCP, collagen gel or HPL gel in culture medium supplemented with 1%, 5%, 10% or 20% HPL. After 7 days proliferation was assessed with the MTT assay (please note that this assay provides a relative measure which may vary between experiments). Overall, cell growth was stimulated with higher concentrations of HPL and it was significantly increased on HPL gel in comparison to TCP and collagen gel (A; n = 7). Furthermore, proliferation of MSC was higher on HPL gel than on fibrin gel (B; n = 3). A similar growth supportive effect of HPL-gel was also observed for dermal fibroblasts (C; n = 4). The Proliferation rate of MSC was also analyzed in the course of culture expansion after 2, 5, 7, and 9 days in 10% HPL (D; n = 3). To quantify initial colony formation on either TCP or HPL gel the cells were seeded in limiting dilution in 96-well plates. After 14 days, the wells were scored for colony formation using MTT assay and the CFU-f frequency was determined by Poisson statistics (E; n = 3) *P<0.05; **P<0.005; ***P<0.0005.
**Figure 8** shows a schematic representation of experimental arrangements (A) and diagrams demonstrating culture expansion and passaging of MSCs in HPL gels (B and C). Scheme of culture expansion on TCP or HPL-gel: MSC on TCP were continuously passaged after reaching a confluency level of 80% with trypsin-EDTA. In parallel, cells on HPL gel were harvested together with the gel by pipetting and reseeding without separation of MSC from their matrix (A). Long-term culture of MSC was performed in parallel with both methods over four consecutive passages. Cumulative population doublings (cPD) were calculated from the first passage onward. Representative data of three independent experiments are shown (B). For comparison of different passaging methods 1,000 MSC were cultured overnight on either TCP or HPL gel. MSC on TCP were then passaged with trypsin whereas the cells on HPL gel were propagated back to TCP by either pipetting, additional treatment with trypsin/EDTA or incubation with plasmin. After one further day adherent and non-adherent cells were measured using the MTT-assay (C).

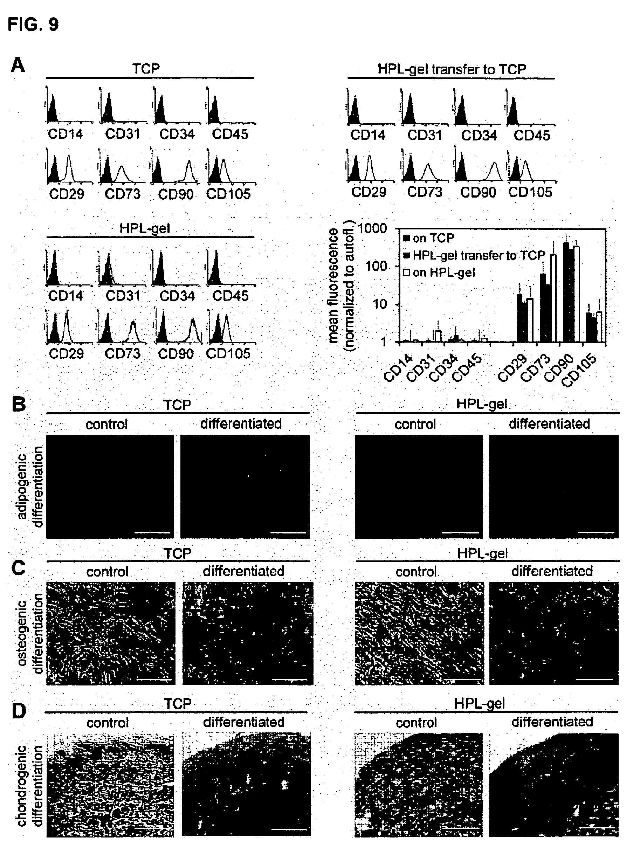

**Figure 9** shows a comparison of MSCs cultured on tissue culture plastic and HPL gel. Cells were isolated and expanded for three passages either on TCP, on HPL gel, or on HPL gel with transfer to TCP for one additional passage (A). Representative histograms are presented for each cell preparation. All MSC revealed the same CD14⁺, CD31⁻, CD34⁻, CD45⁻, CD29⁺, CD73⁺, CD90⁺, and CD105⁺ immunophenotype without significant differences in the mean signal intensity (n = 4; normalized to the corresponding autofluorescence). MSC isolated on either TCP or HPL gel were then induced towards adipogenic (B) or osteogenic (C) lineage on conventional TCP for three weeks, whereas chondrogenic differentiation was induced in pellet culture for three weeks (D). Differentiation was analyzed the green fluorescent dye BODIPY, by Alizarin Red staining, or Alcian blue staining, respectively. Representative data of three independent experiments are shown. Scale bar: 200 µm.

### Description of exemplary and preferred embodiments of the invention

Mesenchymal stromal cells (MSC) are a biologically important cell population that is able to support hematopoiesis, can differentiate along mesenchymal and nonmesenchymal lineages *in vitro,* is capable of suppressing alloresponses, and appear to be nonimmunogenic. These properties suggest emerging roles for mesenchymal stromal cells in cell therapy.

MSC were isolated from the caput femoris upon hip fracture after written consent using guidelines approved by the Ethic Committee of the University of Aachen. Bone fragments were flushed with PBS and mononuclear cells (MNC) subsequently isolated by a density gradient on Biocoll (Biochrom AG, Berlin, Germany). Cells were cultured in fibronectin coated cell culture dishes (Greiner, Kremsmünster, Austria) in Dulbecco's Modified Eagles Medium-Low Glucose (DMEM-LG; PAA, Pasching, Austria) with 2 mM L-glutamine (Sigma Aldrich, St. Louis, MO, USA) and 100 U/mL penicilline/streptomycine (pen/strep; Lonza, Basel, Switzerland). Culture medium was supplemented with FCS (HyClone, Bonn, Germany) or HPL as indicated. Culture medium was always changed twice per week. When reaching 80% confluence cells were trypsinized, counted by in a Neubauer counting chamber (Brand, Wertheim, Germany) and replated at 10⁴ cells/cm². Cumulative population doublings were calculated from the first passage onward.
Alternatively, MSC may also be isolated from lipoaspirates.

Human platelet lysate (HPL) was generated from platelet units of healthy donors as described by Horn et al. (2010). In particular, HPL was generated by apheresis using the Trima Accel collection system (CaridianBCT, Garching, Germany). Such thrombocyte concentrates had a platelet content of 2.0 to 4.2×10¹¹ platelets in 200 mL plasma supplemented with Acid-Citrate-Dextrose (ACD) (1:11 v/v). Platelet units were aliquoted, twice frozen at -80°C, re-thawed at 37°C and centrifuged at 2600 *g* for 30 min at 4°C to remove cell fragments and pooled in equal amounts. The supernatant was filtered through 0.22 µm GD/X PVDF filter device (Whatman, Dassel, Germany), optionally supplemented with 2 U/mL heparin to avoid gelatinization, and stored at -80°C.

Cells were cultured in cell culture dishes (Greiner, Kremsmünster, Austria) in Dulbecoo's Modified Eagles Medium-Low Glucose (DMEM-LG; PAA) with 2 mM L-glutamine (Sigma Aldrich, St. Louis, MO, USA) and 100 U/mL penicillin/streptomycin (pen/strep; Lonza, Basel, Switzerland). Culture medium/substrate was supplemented with FCS (HyClone, Bonn, Germany) or HPL as indicated in the specification. Cells were cultured at 37°C in a humidified atmosphere containing 5% carbon dioxide with medium changes twice per week.

Cell proliferation was evaluated via the Thiazolyl Blue Tetrazolium Bromide (MTT) assay. MSC were seeded on a 96-well plate (3,000 cells/well) in culture medium/substrate supplemented with 0, 1, 5, 10, or 20% of FCS or HPL as indicated in the specification. After 7 days of culture, cells were washed with PBS (PAA) and incubated with 1 mM MTT (Sigma-Aldrich) in PBS for 3.5 h. The excess solution was discarded and crystals were resolved in 4 mM HCl in isopropanol (both from Roth, Karlsruhe, Germany). Optical density (OD) was measured at wave length 590 nm with a Tecan Infinite 2000 plate reader (Tecan Trading, Männedorf, Switzerland).

**Figure 1** shows experimental arrangements for cultivating cells either directly on cell culture plastics (a) or on a gel according to the invention comprising human platelet lysate (HPL) but being devoid of heparin (b, c). In the prior art method according to (a), the cells 1 are directly attached to the tissue culture plastic 2 and cultured in a liquid HPL medium 3 containing heparin. According to this known method, the cells 1 are harvested by enzymatic treatment using Trypsin (I). In the method according to the invention (b, c), the cells are cultured on and within a gel-like substrate 4 which comprises HPL but is free of heparin. The liquid culture medium 3, which covers the substrate 4, contains essentially identical components but additionally includes heparin for inhibiting coagulation. The cells can be harvested either by degradation of the gel-like substrate 4 using plasmin (II) or by fragmentation of the gel-like substrate 4 using a pipet 5 and subsequent reseeding (III). As a result, cell passaging is possible without the need of an enzymatic treatment. As the gel-like substrate 4 is biocompatible and hence provides a physiological environment for the cells, the risk of any undesired influence on the cells by tissue culture plastic 2 is minimized. Further, covering the surface of the gel-like substrate 4 with a liquid medium 3 comprising blood platelet lysate and a gelatinization-inhibiting substance advantageously provides a homogenous environment for the cells and thus ensures optimal cultivation conditions.

**Figure 2** shows the proliferation of mesenchymal stromal cells (MSCs) on different surfaces. MSC were cultured in culture medium comprising different concentrations of human platelet lysate (HPL) and the number of cells was determined after seven days using an MTT assay. The HPL concentration within the gel was respectively consistent with the HPL concentration in the liquid medium. Cell proliferation in the HPL gel according to the invention (black bars) was significantly increased if compared to proliferation on plastics (white bars) or collagen gel (grey bars). The increase in proliferation is directly dependent on the concentration of the medium. Accordingly, human platelet lysate (HPL) significantly increases the proliferation rate of adipose tissue derived MSC. Thus, the method according to the invention enables reliable expansion of cells and efficient minimization of contact inhibition as the cells can grow three-dimensionally within the substrate, i.e. multilayered, while growth factors are evenly available in all directions.

**Figure 3** shows a bar diagram demonstrating the proliferation of mesenchymal stromal cells (MSC) for different cell passaging methods with gel comprising human platelet lysate (HPL). MSC were cultivated for seven days on HPL gels and, for the sake of comparison, on cell culture plastics. Cells were harvested using plasmin treatment or pipetting, with or without subsequent Trypsin/EDTA or EDTA treatment, respectively, and then transferred to a new cell culture dish containing medium without HPL. After one further day of culturing, the number of cells was determined by a MTT assay. The relative proliferation values show that cell passaging by simple pipetting without any enzymatic treatment is an advantageous way of detaching and reseeding the cells since cell damaging by enzymatic digestion is avoided.

**Figures 4** and **5** show diagrams illustrating the proliferation of mesenchymal stromal cells (MSC) derived from bone marrow (BM) or adipose tissue (AT) as a function of Heparin ([U/ml]) and LMWH (Clexane^{®}, [mg/ml]) concentration, respectively. As becomes apparent from these diagrams, the number of cells increases with increasing concentration of the gelatinization-inhibiting substance, i.e. Heparin or Clexane^{®}, until a maximum value is reached. If the concentration of the gelatinization-inhibiting substance within the liquid medium is further increased, the number of cells decreases until no proliferation can be observed at high concentrations of Heparin or LMWH. The best proliferation rates with Heparin can be achieved in the range of 0.1-100 U/ml, preferably 0.2-50 U/ml, particularly preferred 0.2-10 U/ml, most preferred 0.5-10 U/ml. With Clexane^{®} the best proliferation rates may be achieved in the range of 0.001-1 mg/ml, preferably 0.005 -1 mg/ml, in particular 0.005-0.5 mg/ml. With low concentrations of gelatinization-inhibiting substances gelatinization occurs. For example, liquid medium supplemented with 10 % HPL gelatinizes if only less than 0.1 U/ml Heparin or less than 0.001 mg/ml Clexane^{®} are added.

### Example

### Generation of human platelet lysate:

Platelet units were kindly provided by the Institute for Transfusion Medicine, RWTH Aachen University Medical School after their expiry date (5 days after harvesting). Platelet units were generated by apheresis using the Trima Accel Collection System (CaridianBCT, Garching, Germany) and comprise 2.0-4.2 × 10¹¹ platelets in 200 mL plasma supplemented with Acid-Citrate-Dextrose (ACD) (1:11 v/v). Aliquots of 45 mL were twice frozen at -80°C and re-thawed at 37°C and then centrifuged at 2,600 × g for 30 minutes. The supernatant was filtered through 0.2 µm GD/X PVDF filters (Whatman, Dassel, Germany) and stored at -80°C until use. Experiments of this study were performed with three HPL-pools each consisting of lysates of four donors but similar results were also observed using individual donor derived HPLs. HPLs consisted predominately of blood type A but a systematic analysis did not reveal any impact of the blood group. In order to avoid HPL gel formation, 2 U/mL heparin (Ratiopharm, Ulm, Germany) were added as an anticoagulant before addition to culture medium.

### Culture medium and HPL-gel:

Culture medium consisted of Dulbecco's Modified Eagles Medium-Low Glucose (DMEM-LG; PAA, Pasching, Austria) with 2 mM L-glutamine (Sigma Aldrich, St. Louis, MO, USA) and 100 U/mL penicillin/streptomycin (pen/strep; Lonza, Basel, Switzerland) and HPL (10% if not indicated otherwise). Cells were cultured on tissue culture plastic (TCP; Greiner, Kremsmünster, Austria). For gel preparation, HPL containing cell culture medium without heparin was filled in cell culture plates. Due to the calcium comprised in the medium the coagulation cascade was initiated and the HPL-medium gelatinized within one hour at 37°C. The resulting HPL-gel was used as cell culture substrate which was seeded with the same amount of MNC, and overlaid with the same HPL-medium supplemented with heparin. For comparison, gels consisting of 80% collagen G (3 mg/mL collagen type I/III in 12 mM HCl; Biochrome, Berlin, Germany) were used as described before. Furthermore, we used fibrin-gels which were made from lyophilized human fibrinogen powder (F3789 Sigma Aldrich, Deisenhofen, Germany) dissolved in Tris-buffered saline (TBS) with an addition of 3.75 mM CaCl₂ and 3 IU/mL thrombin powder (T1063 Sigma Aldrich) dissolved in sterile water for crosslinking. Cells were cultured at 37°C in 6 well plates or T75 tissue culture flasks (Nunc Thermo Fisher Scientific, Langenselbold, Germany).

### Elasticity measurements of HPL gels:

For elasticity measurements, HPL-gels were formed in 5%, 10% and 20% concentrations using plastic cylinders (14 mm in diameter and 2 mm in height) as mold on top of thin cover slides. After gelatinization as described above, mold cylinders were removed and HPL-gels were overlaid with a cover slide of known mass. Gel thickness was determined using a confocal laser scanning microscope (LSM 510 Meta, Zeiss, Germany) equipped with a long distance 40x Achroplan (NA 0.6, PH 2) objective. Placement of additional cover slides of known weight and subsequent thickness measurement were repeated at least two times for each HPL-gel cylinder. From the measured quantities of gel thickness (h), total mass of cover slide(s) (m), thickness reduction due to loading (Dh), and gel cross-section (A), we calculated the elastic modules of the gel according to E= (m*g)/A*(h/□l) where g denotes the acceleration due to gravitation.

### Isolation of human mesenchymal stromal cells from bone marrow:

MSC were isolated from mononuclear cells (MNC) by plastic adherence. In brief, bone fragments from the *caput femoris* of patients undergoing femoral head prosthesis were flushed with phosphate-buffered saline (PBS). Subsequently, MNC were isolated by Ficoll density gradient centrifugation (Biocoll Separating Solution, density 1.077g/l, Biochrom AG, Berlin, Germany) and washed twice with PBS. At least 5 × 10⁵ MNC were then resuspended in culture medium either on TCP, HPL-gel or collagen-gels in 6-well plates. Cells were cultured at 37°C in a humidified atmosphere with 5% CO₂. The first medium exchange was performed after 48 hours to remove non-adherent cells. Thereafter, half-medium changes were performed twice per week and MSC were further passaged as described below.

### Isolation of human dermal fibroblasts:

Fibroblasts were isolated from skin samples of patients undergoing cosmetic surgery. Initially the cells were expanded in DMEM-LG supplemented with glutamine (PAA), penicillin/streptomycin (PAA) and 10% FCS (Biochrom, Berlin, Germany). Cells at passage 3 to 5 were then transferred to HPL-culture conditions as described above.

### Scanning electron microscopy:

Human MSC/HPL-gel matrix were fixed in 3% glutaraldehyde for at least 1 hour at room temperature, rinsed with PBS and dehydrated by incubating consecutively in 30%, 50%, 70%, 90%, and 100% ethanol for 10 minutes at room temperature. HPL-gels were then critical point dried in liquid CO₂ and sputter-coated with a 30 nm gold layer using an ion sputter coater (LEICA EM SCD 500). Samples were analyzed using an environmental scanning electron microscope at the electron microscope facility, RWTH Aachen University (ESEMXL 30 FEG, FEI, Philips, Eindhoven, Netherlands).

### Histo sections-HE staining:

For histological analysis, HPL-gels with cultured cells at the surface were fixed in 3.7% formaldehyde for 24 hours. The constructs were paraffin embedded, orientated on the perpendicular plane, cut with a rotating microtome at 2 µm thickness (Leica, Wetzlar, Germany) and stained with hematoxilin and eosin according to routine histology protocols.

### Proliferation assay and vitality analysis:

Cell proliferation was assessed via the methyl thiazolyl tetrazolium (MTT) assay. Briefly, MSC of passage 3-6 were seeded in 96-well plates (3,000 cells/cm²) either on TCP, collagen-gel, fibrin-gel or HPL-gels in culture medium supplemented with 1%, 5%, 10% or 20% HPL. After 7 days, cells were washed with PBS and incubated with 1 mM MTT (Sigma Aldrich, St. Louis, MO, USA) for 3.5 hours at 37°C. The excess solution was discarded and formazan crystals were resolved in 4 mM HCl in isopropanol (both from Roth, Karlsruhe, Germany). The absorbance was measured at 590 nm using a Tecan Infinite 200 plate reader (Tecan Trading, Switzerland). Each measurement included four technical replicas. Alternativly, proliferation was estimated by carboxyfluorescein diacetate N-succinimidyl ester (CFSE) staining as described before. Furthermore, nuclear staining or live dead staining was performed with DAPI (4',6-Diamidin-2-phenylindol; Molecular Probes) or with 5 µg/mL fluorescein diacetate (Sigma) and 0.5 µg/mL Propidium iodide (BD Pharmingen) respectively, after 7 days of culture on TCP or HPL-gels and analyzed with a Leica DM IL HC fluorescence microscope.

### Fibroblastoid colony-forming unit (CFU-f) assay:

The CFU-f assay was performed by plating MNC in a limiting dilution series in 96-well plates (330,000; 100,000; 33,000; 10,000; 3,000; and 1,000 cells per well; at least 24 wells per dilution step in each experiment) in parallel on both TCP and HPL-gel. Medium was exchanged after 48 hours for the first time and then twice per week. After 14 days in culture, cells were washed twice with PBS and labeled with MTT. All wells with cell growth scored positive and the CFU-f frequency was determined by Poisson statistics (L-calc; Stem Cell Technologies; Vancouver, Canada).

### Passaging and long-term culture of MSC:

MSC on TCP were continuously passaged after reaching a confluency level of 80% using trypsin-EDTA solution 0.25 % (Sigma Aldrich, St. Louis, MO, USA). Cells on HPL-gel were harvested together with the gel by pipetting. Thereby, the gel-MSC mix was diluted with culture medium and then plated onto fresh HPL-gel coated well plates. To determine the MSC cell number, a fraction of the MSC/HPL-gel mixture was seeded on TCP for 24 hours to facilitate attachment to the plastic surface. The plastic-adherent cells were then trypsinized and the cell number was finally determined using Neubauer counting chamber (Brand, Wertheim, Germany). Cell population doublings per passage (PDP) and cumulative population doublings (cPD) were calculated as known in the art. Alternatively, MSC/HPL-gel was incubated with 20 µg plasmin (stock solution 2.76 mg/mL; Enzyme Research Laboratories, Indiana, USA) for 18 hours at 37°C.

To compare different cell propagation methods MSC were seeded in parallel on TCP and HPL gel (1,000 cells/well) and adhered overnight. Cells on TCP were then passaged with trypsin, whereas MSC on HPL-gel were harvested by pipetting with or without additional incubation with trypsin/EDTA for 5 minutes, or with plasmin as described above. All cells were reseeded on TCP and after 1 day the number of adherent and non-adherent cells was estimated using the MTT assay.

### Immunophenotypic analysis:

For characterization of MSC we have used a panel of surface markers. MSC were isolated and cultured for three passages either on TCP or on HPL-gel. To remove the HPL-gel prior to flowcytometric analysis we have either seeded the cells for one additional passage on TCP or we have harvested the HPL-gel by pipetting and washed the cells once in culture medium. Each cell preparation was stained in parallel with the following monoclonal antibodies: mouse anti-human CD14-APC (clone M5E2), CD29-PE (clone MAR4), CD31-PE (clone WM59), CD34-APC (clone 8G12), CD45-APC (clone HI30), CD73-PE (clone AD2), CD90-APC (clone 5E10) (all BD Bioscience, Heidelberg, Germany) and CD105-FITC (clone MAR-226; ImmunoTools, Friesoythe, Germany). Analysis was performed using a FACS Canto II cytometer (BD Bioscience, Heidelberg, Germany) and the collected data were analyzed with WinMDI 2.9 software (Windows Multiple Document Interface for Flow Cytometry). For statistical comparison of different measurements we have normalized the fluorescence intensities to the corresponding autofluorescence measurements.

### In vitro differentiation:

Freshly isolated MSC were directly seeded on either HPL-gels or TCP and expanded for three passages prior to *in vitro* differentiation. Differentiation towards osteogenic, adipogenic and chondrogenic lineage was then induced as known in the art. This differentiation of HPL-gel-MSC was either performed on HPL-gel or upon transfer to TCP. Chondrogenic differentiation was performed in 3D pellet culture upon centrifugation at 400 × g for 7 min. After three weeks, differentiation was analyzed by Alizarin Red stain (osteogenic differentiation), with the green fluorescent dye BODIPY (4,4-difluoro-1,2,5,7,8-pentamethyl-4-bora-3a,4a-diaza-s-indacene; Molecular Probes, Eugene, USA; adipogenic differentiation), or with Alcian blue and nuclear fast red (for sections of chondrogenic differentiated samples) according to routine histology protocols and analyzed with a Leica DM IL HC fluorescence microscope (Leica, Wetzlar, Germany). Furthermore, adipogenic differentiation was quantified on TCP and HPL-gel by counterstaining of all cells with DAPI and direct counting of non-differentiated in relation to differentiated cells (at least 300 cells per experiment).

### Statistics:

Results are expressed as mean ± standard deviation of independent experiments. To estimate the probability of differences we have adopted the paired two-sided Student's T-test.

### Results:

Translucent gels were formed within 30 to 60 minutes upon addition of HPL to culture medium in the absence of anticoagulants. The resulting HPL-gels had a very soft and viscous consistency. The substrate elasticity was extremely low and increased with higher percentages of HPL (5% HPL: 20 Pa; 10% HPL: 28 Pa; 20% HPL: 32 Pa; **Figure 6A**). These gels were over-layered with the same HPL-medium supplemented with heparin to prevent gelatinization of the liquid phase. When MSC were seeded on HPL-gel, they grew at the interface between HPL-gel and HPL-medium without any contact to the plastic surface. Live/dead staining hardly revealed any non-vital cells after seven days of culture. Histological analysis demonstrated multilayered growth at this interface between gel and medium. Cell densities were much higher in HPL-gel in comparison to two-dimensional growth on tissue culture plastic (TCP; **Figure 6B**). Scanning electron microscopy demonstrated the fibrin scaffold of HPL-gel and supported the notion that MSC covered this matrix densely in a multilayered manner (**Figure 6C-E**). MSC may secrete enzymes which degrade fibrin and therefore we assessed the maintenance of a thin HPL-gel upon culture expansion: within the first 4 days hardly any degradation was observed, whereas after 8 days some HPL-gels were partly degraded and cells got in contact with the plastic surface. This was particularly observed in larger culture wells whereas hardly any degradation occurred in multititer plates.

Proliferation was analyzed by seeding plastic adherent MSC in parallel on TCP, collagen scaffolds, fibrin-gel, and HPL-gel. Culture medium was supplemented with different HPL-concentrations (1%, 5%, 10% and 20%) and the HPL-concentrations in gel and medium were adjusted correspondingly (n = 7). After seven days the MTT-assay revealed that proliferation of MSC increased at higher HPL-concentrations and this is in line with previous observations. Notably, cell expansion was significantly higher on HPL-gel than on TCP, collagen-gel (**Figure 7A**), or fibrin-gel (**Figure 7B**). Similar results were observed with dermal fibroblasts (**Figure 7C**). Additionally, we tested proliferation in the course of culture expansion after 2, 5, 7, and 9 days. The proliferative advantage on HPL-gel became more and more apparent - especially after 9 days when MSC were already confluent on TCP (**Figure 7D**). It needs to be taken into account that the MTT-assay is only an indirect indicator for the cell number by measuring the metabolic activity. However, same tendency was also observed when measuring the residual fluorescence upon staining with CFSE or counting of DAPI-stained nuclei. Thus, proliferation was greatly increased on HPL-gel. Subsequently, we compared initial outgrowth of MSC by seeding mono-nuclear cells from human bone marrow in parallel on TCP and HPL-gel to determine the number of fibroblastoid colony forming units (CFU-f). This analysis was performed with limiting-dilution assays in multiwell-format to exclude effects by proliferation or migratory activity. The average CFU-f frequency on TCP was 88.35 per 10⁶ MNC. Interestingly, the CFU-f frequency was 2.24-fold higher on HPL-gel as compared to TCP (P = 0.02; n = 3; **Figure 7E**). These results indicate that HPL-gel enhances outgrowth of MSC.

Long-term culture and large-scale expansion of cells requires serial passaging steps. This is usually performed by harvesting the cells with trypsin/EDTA by the time when the cells reach a certain level of confluency. HPL-gel is too fragile for such harvesting procedures from the gel-surface. Instead, the fibrin-matrix of HPL-gel was degraded by addition of plasmin to the liquid phase overnight - thereby the fibrin scaffold of the HPL-gel was dissolved and the cells relocated on TCP which then facilitated conventional passaging procedures. However, the semi-fluid consistency of HPL-gel enabled a more convenient way for passaging without intermediate contact of the cells to the plastic surface: the viscous HPL-gel was pipetted together with the cells and reseeded without separation of MSC from their matrix (**Figure 8A**). This method was used over four subsequent passages and the expansion rates were similar as compared to the conventional method on TCP with trypsin (**Figure 8B**). To evaluate the cell recovery rate after passaging we determined how many cells remained associated with non-dissolved HPL-gel plugs using the MTT assay: about 1/3 of the cells remained detached with the pipetting procedure, whereas the majority of cells were capable of plastic adherent outgrowth. The percentage of outgrowing cells was slightly increased by additional treatment of trypsin which might disrupt cell-cell adhesion on the HPL-gel. Notably, cell activity was lower upon treatment with plasmin, which might be due to fact that long-term treatment with this peptidase is detrimental to MSC survival (**Figure 8C**).

Culture conditions may affect the composition of subpopulations and they have direct impact on cellular characteristics. For morphological comparison of culture-isolated MSC preparations on either TCP or on HPL-gel we have therefore transferred both for one passage to the same conventional TCP conditions - no differences in plastic adherent growth or cellular morphology were observed. Immunophenotypic analysis of MSC on HPL-gel was either performed with cells which were directly harvested from HPL-gel or upon transferring the cells to TCP for one additional passage. All MSC preparations (on TCP, HPL-gel, or HPL-gel with transfer to TCP) revealed the same CD14⁺, CD31⁻, CD34⁻, CD45⁻, CD29⁺, CD73⁺, CD90⁺, and CD105⁺ immunophenotype without any significant differences (n = 4; **Figure 9A**). *In vitro* differentiation towards adipogenic and osteogenic lineages was initially compared on TCP to exclude potential effects of the different substrates - no differences were observed between MSC which were isolated on TCP or on HPL-gel with transfer to TCP for one passage (n > 3; **Figure 9B-C**). Furthermore, adipogenic differentiation was directly induced on HPL-gel and there was no significant difference in the percentage of cells with lipid droplets on TCP and HPL-gel (42% vs. 37%; n = 3). Osteogenic differentiation was also induced directly on HPL-gel without apparent differences between TCP and HPL-gel (n = 3). Chondrogenic differentiation was induced in pellet culture upon harvesting the cells directly from TCP or from HPL-gel - analysis of proteoglycan deposition with Alcian blue did not reveal significant differences (**Figure 9D**). These results demonstrate that the modified culture procedures on HPL-gel facilitate isolation of cells with typical MSC characteristics.

Human platelet lysate gel provides a new matrix for culture expansion of MSC. The main constituent of the gel is a fibrin scaffold which normally polymerizes into a mesh-like clot during final stages of the hemostatic coagulation cascade. Fibrinogen derives from the plasma fraction of platelet units and the coagulation cascade is inhibited by chelation of calcium ions with citrate. Upon addition to DMEM-culture medium, the excess of calcium ions stimulates polymerization into a fibrin matrix. This matrix has many advantages: (i) it is naturally occurring - especially in areas of wound formation and regeneration; (ii) it is biocompatible and biodegradable; (iii) it is translucent facilitating microscopic observations; and (iv) there is increasing interest in fibrin scaffolds for tissue engineering applications. Furthermore, HPL-gel may be injected in combination with embedded cells in transplantation settings which might facilitate higher recovery and survival of cells than injection of cell pellets in aqueous solutions.

Efficient expansion and high proliferation rates are important for many cell applications. We demonstrate that proliferation was higher on HPL-gel in comparison to culture on TCP, fibrin-gel or collagen-gel. HPL has been demonstrated to be a very effective serum supplement which further enhances proliferation of MSC in comparison to FCS. The platelet fraction comprises relevant growth factors such as platelet derived growth factors (PDGFs) which support this tremendous growth stimulation in comparison to human plasma or serum. The increased proliferation on HPL-gel might be due to the following reasons: 1) in HPL-gel nutrients and growth factors are accessible to the cells from all directions and their supply is buffered by the underlying matrix; 2) cell growth is not strictly limited to two dimensions with contact inhibition upon confluent growth. Instead the cells proliferate more homogeneously and reach higher cell densities on HPL-gel; and 3) HPL-gel might facilitate better adhesive interaction with specific adhesion proteins such as integrins. This might also contribute to the significantly enhanced outgrowth of CFU-f on HPL-gel. Taken together, HPL-gel strongly enhances expansion of MSC - by a better recovery of initial colonies, faster proliferation rates and higher cell densities in multiple layers.

Propagation of cells over subsequent passages usually involves enzymatic treatment, e.g. with trypsin or Accutase™, in order to harvest the cells from the cell culture surface - after washing steps the cell suspension is then seeded on new culture dishes. This procedure has little impact on cell survival but it stalls them in their proliferative cycle. Another alternative for enzyme-free cell harvest is the use of thermoresponsive polymers which can release cells after a temperature shift. If required, HPL-gel can be dissolved by plasmin which is a physiological peptidase although it has been shown that exogenous plasmin impairs survival of MSC over time. More importantly, the very soft and viscous nature of HPL-gel facilitates an alternative procedure by pipetting cells together with their matrix on new culture plates. This was only possible due to the extremely soft nature of HPL-gel - conventional collagen and fibrin-gels are more rigid. Initially, we expected that cell recovery might be hindered as cells can be trapped in HPL-gel-fragments or they might be disrupted during the pipetting procedure. Despite some cell loss in the HPL-gel fragments the expansion rates were in a similar range as with the conventional methods using trypsin on TCP - probably due to the largely increased proliferation rate. The passaging procedure without separating cells from their matrix has several advantages as washing steps and centrifugation are not required. This is of specific interest for improved protocols for cell culture automation. Automation approaches may help to serve the increasing and costly needs in cellular therapy in the future and a simple pipetting procedure is much easier to implement on such platforms.

It is not a trivial task to determine precise cell numbers on HPL-gel. For most experiments we used the MTT-assay which does not require separation of cells from the gel matrix. However this assay provides only an indirect estimate for cell numbers by measuring the conversion of MTT into formazan. Alternatively, we counted DAPI-stained nuclei but reliable quantification was impaired by the localization of cells in different levels on the HPL-gel. Plasmin can be used to degrade the HPL-gels but this may compromise cell viability over time and correspondingly the MTT-signal was lower after plasmin treatment. Alternatively, we diluted the HPL-gel with culture medium to transfer these cells to conventional TCP surfaces. As described above this passaging procedure worked efficiently and the majority of MSC could transfer to the TCP surface - potentially by migration or thinning of HPL-gel with the pipetting procedure. However about 1/3 of the cells remained non-adherent in floating HPL-fragments. This cell loss could be slightly but not significantly reduced by additional treatment with trypsin/EDTA potentially due to disruption of cell-cell adhesion between MSC on HPL-gel. Another important aspect is the stability of HPL-gel which can be partially degraded by fibrinolytic enzymes secreted by MSC. On the other hand, our study demonstrated that it is possible to use HPL-gel even for long-term culture over 3 weeks without addition of antifibrinolytic agents although the stability might be enhanced by aprotinin or tranexamic acid.

Standardization of protocols is an important issue in generation of therapeutic cell products. Serum supplements such as FCS and HPL notoriously reveal batch-to-batch variation. In our previous work we demonstrated differences in HPL generated from individual platelet units and the stimulatory effect declined significantly with HPL-donor age. It has to be noted that we observed also some variation in the gelatinization process which might result from the plasma-fraction of HPL. Further research will enable better control of gel-formation and standardization of HPL-gels and it will provide better insight in the HPL-donor associated variation. This variation may be leveled by pooling of several HPLs, whereas it may be favorable to use single donor derived HPL-gel - potentially even from the transplant-recipient - to reduce the risk of infections with human pathogens.

Cell growth and *in vitro* differentiation can be influenced by surface elasticity. HPL-gel revealed a substrate elasticity of about 30 Pa - this is extremely low and even below the estimated stiffness of adipose tissue. It might be anticipated that adipogenic differentiation is more pronounced on HPL-gel than on TCP but this effect did not become evident in our analysis. Overall, we did not detect clear effects on morphology, immunophenotype or differentiation potential indicating that the composition of MSC might be similar on HPL-gel and TCP. Either way, HPL-gel facilitates isolation of cells with typical MSC characteristics.

In conclusion, HPL-gel is an extremely soft matrix providing various new perspectives for cell culture. It facilitates cell growth embedded in culture medium components without any contact to artificial biomaterials such as polystyrene. Furthermore, this medium does not include xenogeneic components such as FCS. It is even conceivable to use HPL-gel in an autologous setting to further minimize the risk of immunological effects or infections. The initial outgrowth and the proliferation rate of MSC were much higher on HPL-gel than on TCP. The mechanical consistency allows a simple passaging procedure without the need of separating cells from their matrix or additional washing steps - this convenient propagation procedure is of specific relevance for cell culture automation. HPL-gel may facilitate faster generation of high cell numbers in cellular therapy.

Cell culture in regenerative medicine needs to facilitate efficient expansion according to GMP requirements. Human platelet lysate (HPL) can be used as substitute for fetal calf serum (FCS) without the risk of xenogeneic immune reactions or transmission of bovine pathogens. Heparin needs to be added as anticoagulant before addition of HPL to culture medium - otherwise HPL-medium forms a gel within one hour. Here, we demonstrated that such HPL-gels provide a suitable 3D-matrix for cell culture which - apart from heparin - consists of the same components as the over-layered culture medium. Mesenchymal stromal cells (MSC) grew in several layers at the interface between HPL-gel and HPL-medium without contact to any artificial biomaterials. Notably, proliferation of MSC was much higher on HPL-gel compared to tissue culture plastic (TCP). Furthermore, the frequency of initial fibroblastoid colony forming units (CFU-f) was increased on HPL-gel. The viscous consistency of HPL-gel enabled passaging with a convenient harvesting and re-seeding procedure by pipetting cells together with their HPL-matrix - this method does not require washing steps and can easily be automated. The immunophenotype and *in vitro* differentiation potential towards adipogenic, osteogenic and chondrogenic lineage were not affected by culture-isolation on HPL-gel. Taken together, HPL-gel has many advantages over conventional plastic surfaces: it facilitates enhanced CFU-f outgrowth, increased proliferation rates, higher cell densities and non-enzymatic passaging procedures for culture expansion of MSC.

### Non-patent literature

Blande, I. S.; Bassaneze, V.; Lavini-Ramos, C.; Fae, K. C.; Kalil, J.; Miyakawa, A. A.; Schettert, I. T.; Krieger, J. E.: Adipose tissue mesenchymal stem cell expansion in animal serum-free medium supplemented with autologous human platelet lysate. Transfusion 49: 2680-2685; 2009.
Heiskanen, A.; Satomaa, T.; Tiitinen, S.; Laitinen, A.; Mannelin, S.; Impola, U.; Mikkola, M.; Olsson, C.; Miller-Podraza, H.; Blomqvist, M.; Olonen, A.; Salo, H.; Lehenkari, P.; Tuuri, T.; Otonkoski, T.; Natunen, J.; Saarinen, J.; Laine, J.: N-glycolylneuraminic acid xenoantigen contamination of human embryonic and mesenchymal stem cells is substantially reversible. Stem Cells 25:197-202; 2007.
Horn, P.; Bokermann, G.; Cholewa, D.; Bork, S.; Walenda, T.; Koch, C.; Drescher, W.; Hutschenreuther, G.; Zenke, M.; Ho, A.; Wagner, W.: Comparison of Individual Platelet Lysates for Isolation of Human Mesenchymal Stromal Cells. Cytotherapy 12: 888-898; 2010.
Sundin, M.; Ringden, O.; Sundberg, B.; Nava, S.; Gotherstrom, C.; Le Blanc K.: No alloantibodies against mesenchymal stromal cells, but presence of anti-fetal calf serum antibodies, after transplantation in allogeneic hematopoietic stem cell recipients. Haematologica 92:1208-1215; 2007.

## Claims

1. Method for cultivating adherent cells using a liquid medium comprising blood platelet lysate and a gelatinization-inhibiting substance, said method comprising cultivating the cells on the surface of and/or within a three-dimensional, gel-like substrate comprising 1-40 % blood platelet lysate but being substantially devoid of a gelatinization-inhibiting substance, wherein the liquid medium is layered on the gel-like substrate.

2. Method according to claim 1, wherein the liquid medium and the gel-like substrate, beside the gelatinization-inhibiting substance, have a substantially identical composition.

3. Method according to claim 1 or 2, wherein passaging or harvesting the cells is accomplished by enzymatic degradation of the gel-like substrate, preferably using a peptidase, in particular plasmin.

4. Method according to claim 1, 2 or 3, wherein passaging or harvesting the cells is accomplished by mechanical fragmentation of the gel-like structure, preferably by pipetting.

5. Method according to any one of claims 1 to 4, wherein the gelatinization-inhibiting substance comprises heparin or a low molecular weight heparin (LMWH).

6. Method according to any one of claims 1 to 5, wherein the liquid medium comprises 0.1-100 U/ml Heparin or 0.001-1 mg/ml LMWH as gelatinization-inhibiting substance.

7. Method according to any one of claims 1 to 6, wherein both the liquid medium and the gel-like substrate are substantially devoid of serum.

8. Method according to any one of claims 1 to 7, wherein said cells are stem or progenitor cells, preferably mesenchymal stem cells, in particular mesenchymal stromal cells.

9. Method according to any one of claims 1 to 8, wherein said cells are mesodermal cells, preferably fibroblasts or mesenchymal stromal cells.

10. A method for producing a three-dimensional, gel-like substrate, said method comprising:
- Providing a thrombocyte concentrate;
- Harvesting blood platelets from said concentrate;
- Lysing the blood platelets;
- Adding at least one medium component to the lysate so as to obtain a medium comprising 1-40 % blood platelet lysate; and
- Allowing the blood-platelet-lysate-medium to gelatinize at approximately 37 °C so as to obtain a gel-like substrate comprising blood platelet lysate.

11. Method according to claim 10, wherein said medium component comprises a standardized medium composition for cell culture, preferably DMEM medium.

12. Method according to claim 10 or 11, wherein said substrate comprises 5-40 % blood platelet lysate, more preferred 5-20 % blood platelet lysate, in particular approximately 10- 20 % blood platelet lysate.

13. A two-phase system comprising (a) a liquid medium comprising blood platelet lysate and a gelatinization-inhibiting substance and (b) a three-dimensional, gel-like substrate comprising 1-40 % blood platelet lysate but being substantially devoid of a gelatinization-inhibiting substance.

14. The two-phase system according to claim 13, wherein the liquid medium and the gel-like substrate, beside the gelatinization-inhibiting substance, have a substantially identical composition.

## Patentansprüche

1. Verfahren zur Kultivierung adhärenter Zellen mittels eines flüssigen Mediums, welches Blutplättchen-Lysat und eine die Gelierung hemmende Substanz umfasst, wobei dieses Verfahren das Kultivieren der Zellen auf der Oberfläche und/oder innerhalb eines dreidimensionalen, gelartigen Substrats umfasst, welches 1-40 % Blutplättchen-Lysat umfasst, aber im Wesentlichen frei von einer die Gelierung hemmende Substanz ist, wobei das flüssige Medium über das gelartigen Substrat geschichtet ist.

2. Verfahren nach Anspruch 1, wobei das flüssige Medium und das gelartige Substrat, abgesehen von der die Gelierung hemmenden Substanz, eine im Wesentlichen identische Zusammensetzung haben.

3. Verfahren nach Anspruch 1 oder 2, wobei Passagieren oder Ernten der Zellen durch enzymatischen Abbau der gelartigen Substanz, vorzugsweise mittels einer Peptidase, insbesondere Plasmin, durchgeführt wird.

4. Verfahren nach Anspruch 1, 2 oder 3, wobei Passagieren oder Ernten der Zellen durch mechanische Zerkleinerung der gelartigen Substanz, vorzugsweise durch Pipettieren, durchgeführt wird.

5. Verfahren nach einem der Ansprüche 1 bis 4, wobei die die Gelierung hemmende Substanz Heparin oder niedermolekulares Heparin (LMWH) umfasst.

6. Verfahren nach einem der Ansprüche 1 bis 5, wobei das flüssige Medium 0,1-100 U/ml Heparin oder 0,001-1 mg/ml LMWH als die Gelierung hemmende Substanz umfasst.

7. Verfahren nach einem der Ansprüche 1 bis 6, wobei sowohl das flüssige Medium als auch die gelartige Substanz im Wesentlichen frei von Serum sind.

8. Verfahren nach einem der Ansprüche 1 bis 7, wobei die Zellen Stamm- oder Vorläuferzellen, vorzugsweise mesenchymal Stammzellen, insbesondere mesenchymale Bindegewebszellen, sind.

9. Verfahren nach einem der Ansprüche 1 bis 8, wobei die Zellen mesodermale Zellen, vorzugsweise Fibroblasten oder mesenchymale Bindegewebszellen, sind.

10. Verfahren zur Herstellung eines dreidimensionalen, gelartigen Substrats, wobei das Verfahren umfasst:
- Bereitstellen eines Thrombozyten-Konzentrats;
- Ernten von Blutplättchen aus diesem Konzentrat;
- Lysieren der Blutplättchen;
- Hinzufügen mindestens einer Medium-Komponente zu dem Lysat, um ein Medium zu erhalten, das 1-40 % Blutplättchenlysat umfasst; und
- Ermöglichen der Gelierung des Blutplättchenlysat-Mediums bei ungefähr 37 °C, um ein gelartiges Substrat zu erhalten, das Blutplättchenlysat umfasst.

11. Verfahren nach Anspruch 10, wobei die Medium-Komponente eine standardisierte Medium-Zusammensetzung für Zellkulturen, vorzugsweise DMEM-Medium, umfasst.

12. Verfahren nach Anspruch 10 oder 11, wobei das Substrat 5-40 % Blutplättchenlysat, vorzugsweise 5-20 % Blutplättchenlysat, insbesondere ungefähr 10- 20 % Blutplättchenlysat, umfasst.

13. Zweiphasen-System umfassend (a) ein flüssiges Medium, das Blutplättchenlysat und eine die Gelierung hemmende Substanz umfasst, und (b) ein dreidimensionales, gelartiges Substrat, das 1-40 % Blutplättchenlysat umfasst, aber im Wesentlichen frei von einer die Gelierung hemmenden Substanz ist.

14. Zweiphasen-System nach Anspruch 13, wobei das flüssige Medium und das gelartige Substrat, abgesehen von der die Gelierung hemmenden Substanz, eine im Wesentlichen identische Zusammensetzung haben.

## Revendications

1. Procédé de culture de cellules adhérentes utilisant un milieu liquide comprenant un lysat de plaquettes sanguines et un inhibiteur de gélification, ledit procédé comprenant la mise en culture des cellules à la surface et/ou à l'intérieur d'un substrat de type gel tridimensionnel comprenant 1-40 % d'un lysat de plaquettes mais étant largement dépourvu d'un inhibiteur de gélification, le milieu liquide étant superposé sur le substrat de type gel.

2. Procédé selon la revendication 1, dans lequel le milieu liquide et le substrat de type gel, outre l'inhibiteur de gélification, ont une composition quasiment identique.

3. Procédé selon les revendications 1 ou 2, dans lequel le repiquage ou la collecte des cellules sont effectués grâce à une dégradation enzymatique du substrat de type gel, de préférence à l'aide d'une peptidase, notamment de la plasmine.

4. Procédé selon les revendications 1, 2 ou 3, dans lequel le repiquage ou la collecte des cellules sont effectués grâce à une fragmentation mécanique de la structure de type gel, de préférence par pipetage.

5. Procédé selon l'une quelconque des revendications 1 à 4, dans lequel l'inhibiteur de gélification comprend de l'héparine ou une héparine de bas poids moléculaire (HBPM).

6. Procédé selon l'une quelconque des revendications 1 à 5, dans lequel le milieu liquide comprend 0,1-100 U/ml d'héparine ou 0,001-1 mg/ml d'HBPM comme inhibiteur de gélification.

7. Procédé selon l'une quelconque des revendications 1 à 6, dans lequel le milieu liquide et le substrat de type gel sont largement dépourvus de sérum.

8. Procédé selon l'une quelconque des revendications 1 à 7, dans lequel lesdites cellules sont des cellules souches ou des cellules progénitrices, de préférence des cellules souches mésenchymateuses, notamment des cellules stromales mésenchymateuses.

9. Procédé selon l'une quelconque des revendications 1 à 8, dans lequel lesdites cellules sont des cellules mésodermiques, de préférence des fibroblastes ou des cellules stromales mésenchymateuses.

10. Procédé destiné à la production d'un substrat de type gel tridimensionnel, ledit procédé comprenant :
- l'utilisation d'un concentré de plaquettes ;
- la collecte de plaquettes à partir dudit concentré ;
- la lyse des plaquettes ;
- l'ajout d'au moins un composant de milieu au lysat dans le but d'obtenir un milieu comprenant 1-40 % d'un lysat de plaquettes ; et
- la gélification du milieu contenant le lysat de plaquettes à 37 °C environ afin d'obtenir un substrat de type gel contenant le lysat de plaquettes.

11. Procédé selon la revendication 10, dans lequel ledit composant de milieu comprend une composition de milieu standardisée pour la culture cellulaire, de préférence un milieu DMEM.

12. Procédé selon les revendications 10 ou 11, dans lequel ledit substrat comprend 5-40 % d'un lysat de plaquettes, de préférence 5-20 % d'un lysat de plaquettes, plus particulièrement 10-20 % environ d'un lysat de plaquettes.

13. Système biphasique, comprenant (a) un milieu liquide contenant un lysat de plaquettes et un inhibiteur de gélification et (b) un substrat de type gel tridimensionnel comprenant 1-40 % d'un lysat de plaquettes, mais étant largement dépourvu d'un inhibiteur de gélification.

14. Système biphasique selon la revendication 13, dans lequel le milieu liquide et le substrat de type gel, outre l'inhibiteur de gélification, ont une composition quasiment identique.
